# EUROPÄISCHE PATENTSCHRIFT

(11) **EP 3 228 250 B1**
(45) Veröffentlichungstag und Bekanntmachung des Hinweises auf die Patenterteilung: **25.08.2021**
(21) Anmeldenummer: 17161235.1
(22) Anmeldetag: 16.03.2017
(51) Int. Cl.: A61B 6/06, A61B 6/00

(54) **VORRICHTUNG UND VERFAHREN ZUR ERSTELLUNG EINER RÖNTGENPANORAMAAUFNAHME**
METHOD AND DEVICE FOR CREATING AN X-RAY PANORAMIC IMAGE
DISPOSITIF ET PROCÉDÉ DE FABRICATION D'UNE RADIOGRAPHIE PANORAMIQUE

(30) Priorität: 30.03.2016 DE 102016205176
(43) Veröffentlichungstag der Anmeldung: 11.10.2017
(73) Patentinhaber: Siemens Healthcare GmbH, 91052 Erlangen (DE)
(72) Erfinder: Ecabert, Olivier, 91320 Ebermannstadt (DE); Gemmel, Alexander, 91056 Erlangen (DE); Kleinszig, Gerhard, 91301 Forchheim (DE); Tamersoy, Birgi, 91052 Erlangen (DE)

(56) Entgegenhaltungen:
- EP-A1- 1 484 016
- WO-A1-96/15722
- US-A1- 2014 219 420

## Beschreibung

Die Erfindung bezieht sich auf eine Vorrichtung bzw. ein Verfahren zur Zusammensetzung einer aus mehreren Einzelröntgenbildern bestehenden Gesamt- bzw. Röntgenpanoramaaufnahme.

Unterstützend für eine ärztliche Diagnose können z.B. Röntgenbilder angelegt werden. Mit den Röntgenbildern können unter anderem Gefäß- und Knochenstrukturen sowie Ausrichtungen der Wirbelsäule oder der Beine visualisiert werden. Lange Röhrenknochen, die gesamte Wirbelsäule oder auch die Abbildung eines Beines mit Hüftgelenk einschließlich des Oberschenkelknochens, des Knie- und des Sprunggelenks, z.B. zur Bestimmung einer Beinachse, sind mit einem einzelnen Röntgenbild, wegen einer die Röntgenbildgröße limitierenden Detektorgröße, nur sehr schwer bestimmbar. Um eine Gesamtbetrachtung z.B. der Wirbelsäule, eines Beines oder eines Armes zu erhalten, werden eine Vielzahl von aneinander zu reihenden Röntgenbildern, nachfolgend auch als Teilröntgenbilder bezeichnet, von dem zu betrachtenden Körperabschnitt angelegt. Zur Erstellung eines Gesamtbildes werden jeweils überlappende Bereiche bei den aneinander zu fügenden Teilröntgenbildern vorgesehen, um anhand von übereinstimmenden markanten Strukturen in den überlappenden Bereichen der angrenzenden Teilröntgenbilder aneinanderzufügen. Sind diese markanten Strukturen nur eingeschränkt vorhanden, so können mittels Korrelationsverfahren Übereinstimmungen errechnet und die angrenzenden Röntgenbilder zueinander ausgerichtet werden. Um beispielsweise Korrelationsverfahren erfolgreich durchzuführen, müssen aber geeignet große überlappende Bereiche vorhanden sein. Breite überlappende Bereiche bringen aber für den Patienten den Nachteil mit sich, dass er einer erhöhten Röntgendosis ausgesetzt ist. Darüber hinaus bedarf es aufwändiger zeitintensiver Rechenoperationen, um Übereinstimmungswahrscheinlichkeiten in überlappenden Bildanteilen zu ermitteln. Ein Beispiel für die beschriebenen Röntgenanlagen ist in der WO 96/15722 gezeigt. Diese Vorrichtung beinhaltet eine Röntgenquelle, eine Röntgenblendeneinheit mit einem Kollimator und einen Detektor zum Anlegen von Teilröntgenbildern, wobei der Kollimator verfahrbar ist.

Der Erfindung liegt die Aufgabe zugrunde, eine weitere Vorrichtung und ein dazugehöriges Verfahren zur Erstellung einer aus Teilröntgenbildern zusammen zu fügenden Röntgenpanoramaaufnahme anzugeben.

Die der Erfindung zugrundeliegende Aufgabe wird durch die im Patentanspruch 1 oder 5 angegebenen Merkmale gelöst.

Die Vorrichtung und das dazugehörige Verfahren weist eine Röntgenanlage zum Anlegen von Teilröntgenbildern für eine Röntgenpanoramaaufnahme auf. Diese Röntgenanlage ist mit einer Röntgenblendeneinheit mit einem ersten und/oder zweiten semitransparenten Kollimator ausgestattet. Der erste und/oder zweite semitransparente Kollimator kann beim Anlegen von Teilröntgenbildern für eine Röntgenpanoramaaufnahme derart angesteuert werden, dass die Kollimatoren in die Blendenöffnung der Röntgenblendeneinheit derart gefahren werden, dass zumindest Teile der sich überlappenden Bereiche von den aneinanderzufügenden Teilröntgenbildern mit einer reduzierten Röntgendosis anlegbar sind, wobei die Breite eines Überlappungsbereichs zwischen zwei angrenzenden Teilröntgenbildern vorgebbar ist.

Die Erfindung bringt den Vorteil mit sich, dass die Röntgenstrahlenbelastung zur Erstellung einer Röntgenpanoramaaufnahme für den Patienten erheblich reduziert ist.

Die Erfindung bringt den Vorteil mit sich, dass die Röntgenpanoramaaufnahme schnell, präzise und reproduzierbar erstellt werden kann.

Die Erfindung bringt den Vorteil mit sich, dass die überlappenden Teile von aneinandergrenzenden Teilröntgenbildern mit einer erheblich reduzierten Röntgendosis und Bereiche mit hoher Aussagekraft mit einer hierfür optimierten Röntgendosis anlegbar sind.

Die Erfindung bringt den Vorteil mit sich, dass die einzelnen Teilröntgenbilder ohne sichtbare Übergänge aneinanderfügbar sind.

Die Erfindung bringt den Vorteil mit sich, dass das Ausrichten der einzelnen Teilröntgenbilder einer Röntgenpanoramaaufnahme anhand von Merkmalen in parallaxenfreien Bereichen in den einzelnen Teilröntgenbildern erfolgt.

Vorteilhafte Ausgestaltungen der Erfindung sind in den Unteransprüchen angegeben.

Die Erfindung soll im Folgenden mittels der in den Zeichnungen dargestellten Ausführungsbeispiele näher erläutert werden.

Es zeigen:
- Figur 1: eine schematische Anordnung einer Röntgenanlage,
- Figur 2: das Entstehen einer ersten Röntgenpanoramaaufnahme und
- Figur 3: das Entstehen einer zweiten Röntgenpanoramaaufnahme.

Durch die Vorrichtung und das dazugehörige Verfahren wird bei der Erstellung einer Röntgenpanoramaaufnahme die Röntgenbelastung für den Patienten trotz größerer Überlappungsbereiche beim Anlegen von Teilröntgenbildern durch eine Verwendung von semitransparenten Röntgenblenden reduziert.

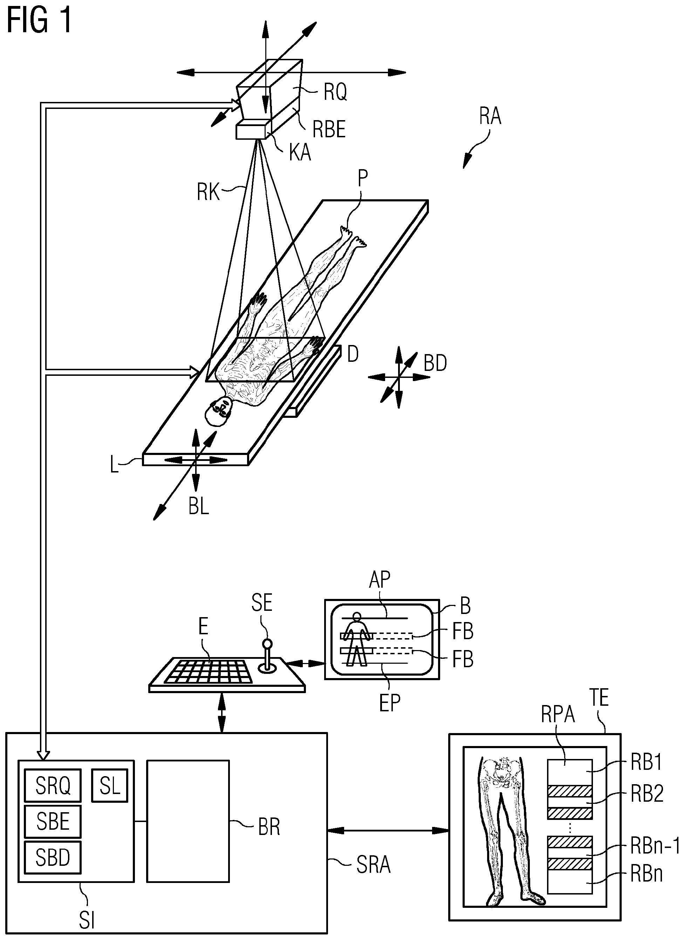

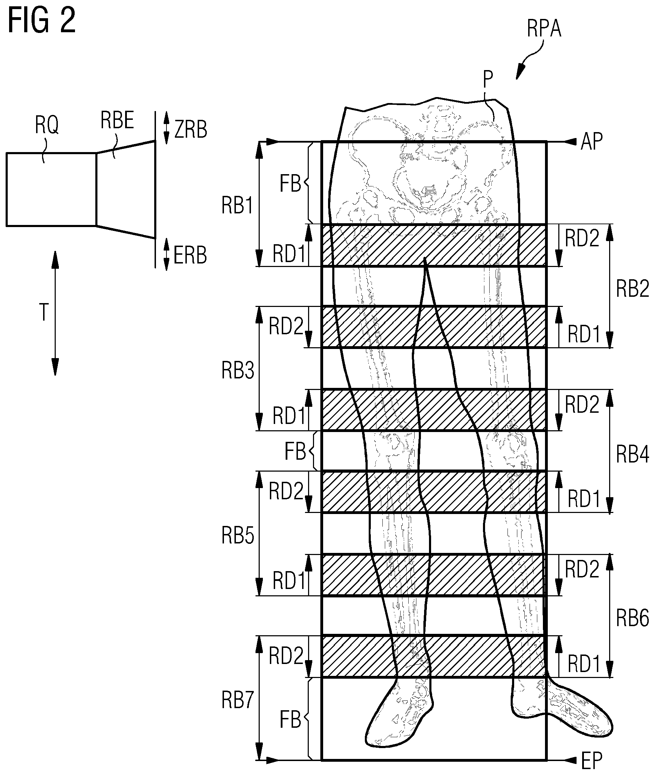

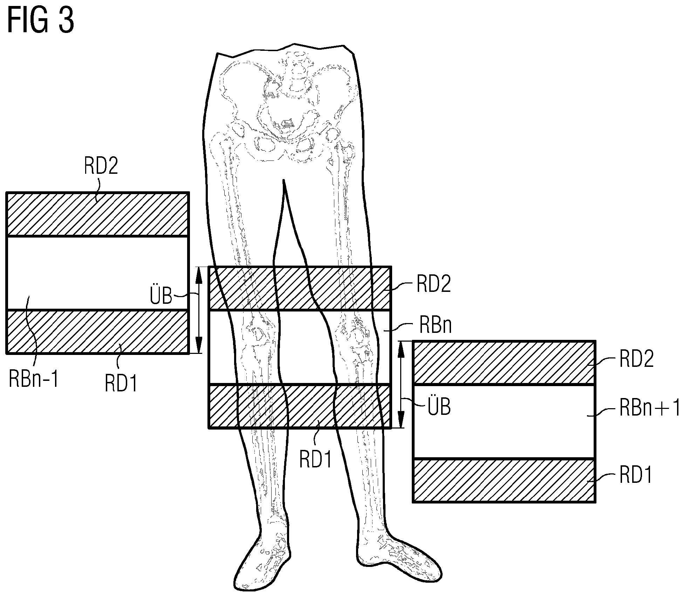

In Figur 1 ist eine Röntgenanlage RA vereinfacht abgebildet. Diese Abbildung zeigt schematisch einen auf einer Liege L platzierten Patienten P. Von diesem Patienten P soll beispielsweise eine Röntgenpanoramaaufnahme ab der Hüfte mit den Beinen erstellt werden. Oberhalb der Liege L ist eine Röntgenquelle RQ mit einer Röntgenblendeneinheit RBE sowie unterhalb der Liege ein Detektor D angeordnet. An der Röntgenblendeneinheit RBE ist eine Aufzeichnungseinheit, beispielsweise eine Kamera KA, befestigt. Im Falle einer starren Verbindung zwischen Röntgenquelle RQ und Detektor D wird diese Anordnung Decken- oder Bodengestützt zum Anlegen eines Röntgenpanoramabildes RPA entlang des Patienten P bestimmungsgemäß verfahren. Im Falle einer statischen Anordnung von Röntgenquelle RQ und Detektor D kann die Liege L mit dem auf ihr positionierten Patienten P verfahren werden. Die Bewegungsrichtungen der einzelnen Komponenten sind angedeutet. Bei selbständigen Systemen mit jeweils einzelnen Führungssystemen für die Röntgenquelle RQ und den Detektor D können diese aufeinander abgestimmt und die Röntgenquelle RQ und der Detektor D zueinander ausgerichtet entlang des Patienten P verfahren werden. Kombinierte bzw. korrespondierende Bewegungen zwischen Röntgenquelle RQ, Detektor D, Liege L sind ebenfalls möglich um eine Röntgenpanoramaaufnahme RPA vom Patienten P zu erstellen. Die jeweiligen Positionen von Röntgenquelle RQ, Detektor D und Liege L und Patient P sind u.a. dem Bildrechner BR zur Erstellung der Übersichtsaufnahme bzw. der Röntgenpanoramaaufnahme bekannt. Die einzelnen hier abgebildeten Komponenten wie Röntgenquelle RQ, Röntgenblendeneinheit RBE, Kamera KA, Detektor D, Liege L und Patient P sind so ausgebildet, das deren Positionen bei jedem Röntgenbild bzw. Teilröntgenbild für eine Röntgenpanoramaaufnahme an die Röntgenanlagensteuereinheit SRA weitergeleitet werden. Auf diese Positionsdaten kann der in der Röntgenanlagensteuereinheit SRA angeordnete Bildrechner BR zugreifen. Die Ausrichtung beziehungsweise das Verfahren der einzelnen Komponenten wie Röntgenquelle RQ, Detektor D, oder evtl. Liege L kann mit einer einer Eingabeeinheit E zugeordneten Steuereinheit SE erfolgen. In der Röntgenanlagensteuereinheit SRA sind u.a. einzelne prozessorgesteuerte Steuereinheiten wie eine Röntgensteuereinheit SRQ, eine Blendensteuereinheit SBE, eine Detektorsteuereinheit SBD und eine Liegensteuereinheit SL angeordnet. Die Röntgenquelle RQ wird über eine mit in der Röntgenanlagensteuereinheit SRA angeordneten Röntgenquellensteuereinheit SRQ mit den anderen Komponenten der Röntgenanlage RA koordinierend gesteuert. Die Röntgenblendeneinheit RBE kann über die Blendensteuereinheit SBE derart angesteuert werden, dass zumindest eine erste und/oder zweite semitransparente Röntgenblende ERB, ZRB innerhalb der Röntgenblendeneinheit RBE derart ansteuerbar ist, dass diese entsprechend der Position eines Teilröntgenbildes RB1, ...,RBn innerhalb der Röntgenpanoramaaufnahme RPA in den von der Röntgenquelle RQ ausgehenden Röntgenstrahlkegel RK fahrbar sind. Eine Röntgenpanoramaaufnahme RPA kann dabei, wie in den Figuren 3 oder 2 dargestellt, mit Teilröntgenbildern RB1, ...,RBn unter Verwendung einer einheitlichen oder unterschiedlichen Röntgendosis, insbesondere in den sich überlappenden Bereichen, zusammengesetzt werden. Über die Eingabeeinheit E kann das Anlegen einer Röntgenpanoramaaufnahme RPA menügesteuert, beispielsweise angezeigt an einen Bildschirm B oder über Eingabehilfen einer Touchsreen-Einheit TE, erfolgen. Auf dem Bildschirm B oder der Touchsreen-Einheit TE wird an einen schematisch oder mittels Videocamera abgebildeten Patienten P eine erste Begrenzungslinie AP für den Beginn und eine zweite Begrenzungslinie EP für das Ende des Röntgenpanoramabildes RPA eingegeben. Zusätzlich sind sogenannte Fokusbereiche FB festlegbar in denen das jeweilige Teilröntgenbild RBx mit einer bestmöglichen Röntgenbildqualität erstellt werden soll. Der in der Röntgenanlagensteuereinheit SRA angeordnete Bildrechner BR ermittelt danach die Anzahl der Teilröntgenbilder RB1,..., RBn zwischen der ersten und zweiten Begrenzungslinie AP, EP unter Berücksichtigung von Fokusbereichen FB bei Verwendung eines wählbaren Detektors D bestimmter Größe. Die Breite der möglichen Überlappungsbereiche ÜB der übereinander zu fügenden Randbereiche der Teilröntgenbilder RB1,...,RBn ist ebenso vorgebbar. Danach wird durch Eingabe bestimmt, ob die Röntgenpanoramaaufnahme RPA mit einer gleichbleibenden Röntgenbildqualität oder mit unterschiedler Röntgenbildqualität erstellt werden soll. Um die Röntgenbelastung für den Patienten P signifikant zu reduzieren wird zumindest in den Überlappungsbereichen eine erste und/oder zweite semitransparente Röntgenblende ERB, ZRB in den von der Röntgenquelle RBE ausgehenden Röntgenstrahlkegel RK gefahren. Soll eine Röntgenpanoramaaufnahme RPA mit unterschiedlicher Röntgenbildqualität zusammengestellt werden, so kann ein schmälerer Überlappungsbereich ÜB vorgegeben und dieser jeweils durch die erste und/oder zweite semitransparente Röntgenblende ERB, ZRB abgedeckt werden. Soll eine Röntgenpanoramaaufnahme RPA mit gleichbleibender Röntgenbildqualität gebildet werden, so wird ein großer Überlappungsbereich ÜB vorgegeben und eine semitransparente Röntgenblende ERB, ZRB deckt beispielsweise nur die Hälfte des jeweiligen Überlappungsbereichs ÜB der aneinanderzufügenden Teilröntgenbilder RB1,...,RBn ab.

In Figur 2 ist eine erste Ausführungsvariante zum Anlegen einer Röntgenpanoramaaufnahme RPA gezeigt. Die Röntgenquelle RQ wird entlang einer Trajektorie T verschoben. Mit dieser Röntgenpanoramaaufnahme RDA sollen die Hüfte und die Beine des Patienten P abgebildet werden. Der Beginn der Röntgenpanoramaaufnahme RDA wird mit einer ersten Begrenzungslinie AP am oberen Rand der Hüfte und das Ende der Röntgenpanoramaaufnahme RDA mit einer zweiten Begrenzungslinie EP unterhalb des Sprunggelenks markiert. Angedeutet ist hier der Übersichthalber nur die Röntgenquelle RQ und eine der Röntgenquelle RQ nachgeordnete Röntgenblendeneinheit RBE. Am Ausgang der Röntgenblendeneinheit RBE kann ein erster semitransparenter Kollimator ERB und ein zweiter semitransparenter Kollimator ZRB in die Blendenöffnung der Röntgenblendeneinheit RBE je nach Anforderung in den von der Röntgenquelle RQ abgegebenen Röntgenstrahlkegel RK gefahren werden. Wie weit jeweils der erste und zweite semitransparente Kollimator ERB, ZRB in die anzulegenden Teilröntgenbilder RB1,...RBn geschoben bzw. gefahren wird kann vor Beginn der Röntgenpanoramaaufnahme RPA durch Angabe eines ersten und zweiten Überlappungsbereichs RD1, RD2 vorgegeben werden. Wird beispielsweise mit einem den Hüftbereich des Patienten abbildenden Teilröntgenbild RB1 begonnen, so wird der erste semitransparente Kollimator ERB in der Röntgenblendeneinheit RBE nach Vorgabe der Größe eines ersten Überlappungsbereiches RD1 in den von der Röntgenquelle RQ abgegebenen Röntgenkegel gefahren. Für ein zweites Teilröntgenbild RB2 wird die Röntgenquelle RQ entlang der Trajektorie T entsprechend verfahren. Während des Verfahrens der Röntgenquelle RQ wird die zweite semitransparente Kollimator ZRB in die Öffnung der Röntgenblendeneinheit RBE gefahren. Das zweite Teilröntgenbild RB2 beginnt dort wo der erste Überlappungsbereich RD1 im ersten Teilröntgenbild RB1 beginnt. Das zweite Teilröntgenbild RB2 weist im Gegensatz zum ersten Teilröntgenbild RB1 einen ersten und zweiten Überlappungsbereich RD1, RD2 auf. Innerhalb dieser ersten und zweiten Überlappungsbereiche RD1, RD2 ist die Röntgendosis der auf den Pateinten P gerichteten Röntgenstrahlen entsprechend der verwendeten semitransparenten Röntgenblenden ERB, ZRB reduziert. Die Breite des durch die zweite semitransparente Röntgenblende ZRB erzeugten zweiten Überlappungsbereichs RD2 in dem zweiten Teilröntgenbild RB2 entspricht der Breite des mit reduzierter Röntgendosis aufgenommenen ersten Überlappungsbereich RD1 im ersten Teilröntgenbild RB1. Nachfolgende Teilröntgenbilder RB3,.... RBn-1 werden entsprechend angelegt. Der Bereich zwischen den ersten und zweiten Übergangsbereich RD1, RD2 in den Teilröntgenbildern wird jeweils mit einer auf den jeweiligen Objektabschnitt optimierten Röntgendosis durchleuchtet. Das Anlegen weiterer sich überlappender Teilröntgenbilder RBn für die Röntgenpanoramaaufnahme RPA wird bis zur zweiten Begrenzungslinie EP fortgesetzt. Bei dem letzten Teilröntgenbild RB7 für die Röntgenpanoramaaufnahme RPA wird jedoch der erste semitransparente Kollimator ERB aus der Röntgenblendeneinheit RBE gefahren, da nur noch ein Überlappungsbereich zum vorhergehenden Teilröntgenbild RB6 benötigt wird. Im Gesamtbild ergibt sich eine Röntgenpanoramaaufnahme RPA, das streifenförmig angelegt sein könnte. Diese Röntgenpanoramaaufnahme RPA weist dann Bereiche mit einer kontrastreichen Abbildung zwischen den sich jeweils überlappenden ersten und zweiten Überlappungsbereichen RD1, RD2 auf. Die Teilröntgenbilder RB2, RB3, RB5 sowie RB6 in denen weder Hüftgelenke, Knie oder Sprunggelenke abgebildet sind können mit einer geringeren Röntgendosis angelegt werden. Diese Zusammenstellung des Röntgenpanoramabildes RPA bringt den Vorteil mit sich, das zumindest Bereiche im Ansatz des Beines wie beispielsweise das Pfannengelenkt im Becken, das Knie und das Sprunggelenk des Fußes als Fokusbereiche FB definiert wurden und mit optimierter Röntgenstrahlung in den Teilröntgenbildern RB1, RB4 und RB7 abgebildet werden. Die dazwischenliegenden Röhrenknochen sind für eine Beurteilung oder Ermittlung beispielsweise einer Beinachse von nachgeordneter Bedeutung. In diesen Abschnitten hier wird der Patient P lediglich durch eine reduzierte Röntgendosis belastet.

In Figur 3 ist ein weiteres Ausführungsbeispiel zur Zusammenfügung von einem ersten, zweiten und dritten Teilröntgenbild RBn-1, RBn, RBn+1 zu einer Röntgenpanoramaaufnahme RPA abgebildet. Bei dieser Ausführungsvariante werden, im Unterschied zu dem Ausführungsbeispiel unter Fig. 2, jeweils größere Überlappungsbereiche ÜB bei den Teilröntgenbildern vorgesehen. Diese Überlappungsbereiche ÜB zwischen zwei Teilröntgenbildern weisen mindestens die Breite der durch die ersten und zweiten Röntgenblenden ERB, ZRB erzeugten ersten und/oder zweiten Bereiche RD1, RD2 in den entsprechenden Teilröntgenbildern auf. Wird zu einem Teilröntgenbild RBx ein angrenzendes Teilröntgenbild RBx-1 und/oder RBn+1 zu einer Röntgenpanoramaaufnahme RPA angefügt, so werden die Überlappungsbereiche ÜB zwischen den Teilröntgenbildern RBx-1, RBx und RBx+1 übereinander gelegt. Bei einem Zusammenfügen von Teilröntgenbildern RB1, RB2; RP2, RP3; ...,RBn, RBn+1 reicht der Überlappungsbereich ÜB beispielsweise annähernd jeweils bis zur Mitte der Teilröntgenbilder. In den Überlappungsbereichen liegen jeweils ein Abschnitt eines Teilröntgenbildes mit einer objektbezogenen Röntgenbestrahlung und ein Bereich des Teilröntgenbildes in dem die Röntgenstrahlung durch die Verwendung von einer ersten oder zweiten semitransparenten Röntgenblende ERB, ZRB reduziert wurde übereinander. Betrachtet man beispielsweise den Überlappungsbereich ÜB zwischen dem zweiten und dritten Teilröntgenbild RBn, RBn+1, so liegen ein Abschnitt mit einer optimierten Röntgenbestrahlung aus dem zweiten Teilröntgenbild RBn mit dem zweiten Bereich RD2 aus dem dritten Teilröntgenbild RBn+1 sowie der erste Bereich RD1 aus dem zweiten Teilröntgenbild RBn mit einen Abschnitt des dritten Teilröntgenbildes RBn+1 übereinander. Eine Ausrichtung des zweiten und dritten Teilröntgenbildes RBn, RBn+1 sowie eine Ausrichtung des ersten und zweiten Teilröntgenbildes RBn-1, RBn erfolgt jeweils anhand von identischen markanten Punkten oder Flächenstrukturen in den Überlappungsbereichen ÜB. Nach Abschluss der Korrelation zwischen den jeweils angrenzenden Teilröntgenbildern RB1,...,RBn werden die Bereiche, die mit einer reduzierten Röntgendosis aufgenommen wurden, von den jeweiligen Teilröntgenbildern getrennt und die verbleibenden Teile der Teilröntgenbilder RBn-1, RBn, RBn+1 übergangslos aneinander gefügt.

In einer weiteren Ausführungsvariante kann jeweils nur ein Randbereich pro Teilröntgenbild, beispielsweise ein Bereich der durch eine erste Röntgenblende ERB mit einer reduzierten Röntgendosis RD1 aufgenommen wurde, mit einem Randbereich eines angrenzenden Teilröntgenbildes überlagert werden.

### Bezugszeichenliste

- T: Trajektorie
- RA: Röntgenanlage
- KA: Kamera
- RQ: Röntgenquelle
- RK: Röntgenstrahlkegel
- RBE: Röntgenblendeneinheit
- D: Detektoreinheit
- L: Liege
- P: Patient
- BD: Bewegungsrichtung Detektor
- BL: Bewegungsrichtung der Liege
- SRA: Röntgenanlagensteuereinheit
- S: Steuereinheit
- SL: Liegensteuereinheit
- SRQ: Röntgenquellensteuereinheit
- SBE: Röntgenblendensteuereinheit
- SBD: Detektorsteuereinheit
- BR: Bildrechner
- SE: Joystick
- E: Eingabeeinheit
- TE: Touchscreen-Einheit
- RB1,...,RBn: erstes, ..., n-tes Teilröntgenbild (RBn-x, ..., RBn, ..., RBn+y)
- ERB: erste semitransparente Röntgenblende/Kollimator
- ZRB: zweite semitransparente Röntgenblende/Kollimator
- DA1,..., DAx: Detektorabbildungen
- RPA: Röntgenpanoramaaufnahme
- ÜB: Überlappungsbereich
- B: Bildschirmeinheit
- AP: erste Begrenzungslinie
- EP: zweite Begrenzungslinie
- FB: Fokusbereiche
- RD1: erster Überlappungsbereich
- RD2: zweiter Überlappungsbereich

## Patentansprüche

1. Vorrichtung mit einer eine Röntgenblendeneinheit (RBE) aufweisenden Röntgenquelle (RQ) und einem Detektor (D) zum Anlegen von Teilröntgenbildern (RBn-x,...,RBn,...,RBn+y) wobei die einen ersten und/oder zweiten semitransparenten Kollimator (ERB, ZRB) aufweisende Röntgenblendeneinheit (RBE) beim Anlegen von Teilröntgenbildern (RBn-x,...,RBn,...,RBn+y) einer Röntgenpanoramaaufnahme (RPA) derart ansteuerbar ist,
dass beim Anlegen von Teilröntgenbildern (RBn-x,...,RBn,..., RBn+y) für eine Röntgenpanoramaaufnahme (RPA) der erste und/oder zweite semitransparente Kollimator (ERB, ZRB) in die Blendenöffnung der Röntgenblendeneinheit (RBE) derart verfahren werden, dass zumindest Teile der sich überlappenden Bereiche von aneinanderzufügenden Teilröntgenbildern (RBn-x,..., RBn, ...,RBn+y) mit einer reduzierten Röntgendosis anlegbar sind, wobei die Breite eines Überlappungsbereichs (ÜB) zwischen zwei angrenzenden Teilröntgenbildern (RBn-1,RBn; RBn,RBn+1) vorgebbar ist.

2. Vorrichtung nach Patentanspruch 1,
**dadurch gekennzeichnet,**
**dass** eine Eingabeeinheit (E, B, TE) vorgesehen ist mit der eine erste Begrenzungslinie (AP) und eine zweite Begrenzungslinie (EP) an einem auf einem Bildschirmelement (B, TE) abgebildeten Patienten (P) markierbar sind.

3. Vorrichtung nach Patentanspruch 2,
**dadurch gekennzeichnet,**
**dass** mindestens ein Fokusbereich (FB) vorgebbar ist.

4. Vorrichtung nach einem der vorhergehenden Patentansprüche, **dadurch gekennzeichnet,**
**dass** diese einen Bildrechner (BR) aufweist, wobei die Aufteilung der Teilröntgenbilder (RBn-x,...,RBn,...,RBn+y) für eine Röntgenpanoramaaufnahme (RPA) anhand einer den Patienten (P) aufzeichnenden Kamera (KA) angebbar ist.

5. Verfahren zum Betreiben einer Vorrichtung mit einer eine Röntgenblendeneinheit (RBE) aufweisenden Röntgenquelle (RQ) und einem Detektor (D) zum Anlegen von Teilröntgenbildern (RBn-1, RBn, ...,RBn+1) wobei beim Anlegen von Teilröntgenbildern (RBn-1, RBn, ..., RBn+1) für eine Röntgenpanoramaaufnahme (RPA) ein erster und/oder zweiter semitransparenter Kollimator (ERB, ZRB) in die Blendenöffnung der Röntgenblendeneinheit (RBE) derart verfahren wird, dass zumindest Teile der sich überlappenden Bereiche von aneinanderzufügenden Teilröntgenbildern (RBn-1, RBn, ...,RBn+1) mit einer reduzierten Röntgendosis angelegt werden, und die Breite der Überlappungsbereiche (ÜB) zwischen zwei angrenzenden Teilröntgenbildern (RBn-1,RBn; RBn,RBn+1) vorgegeben wird.

6. Verfahren nach Patentanspruch 5,
**dadurch gekennzeichnet,**
**dass** eine erste Begrenzungslinie (AP) und eine zweite Begrenzungslinie (EP) an einem auf einem Bildschirmelement (B, TE) abgebildeten Patienten (P) markierbar sind.

7. Verfahren nach einem der Patentansprüche 5 oder 6,
**dadurch gekennzeichnet,**
**dass** mindestens ein Fokusbereich (FB) angegeben wird.

8. Verfahren nach einem der vorhergehenden Patentansprüche 5 bis 7,
**dadurch gekennzeichnet,**
**dass** die Aufteilung der Teilröntgenbilder (RBn-x,...,RBn,...,RBn+y) für eine Röntgenpanoramaaufnahme (RPA) anhand einer den Patienten (P) aufzeichnenden Kamera (KA) angelegt wird.

## Claims

1. Device with an x-ray source (RQ) having an x-ray screen unit (RBE) and a detector (D) for creating partial x-ray images (RBn-x,..., RBn,..., RBn+y),
wherein
the x-ray screen unit (RBE) having a first and/or second semi-transparent collimator (ERB, ZRB) can be actuated when partial x-ray images (RBn-x,..., RBn,..., RBn+y) of a panoramic x-ray image (RPA) are created such that when partial x-ray images (RBn-x,..., RBn,..., RBn+y) are created for a panoramic x-ray recording (RPA), the first and/or second semi-transparent collimator (ERB, ZRB) are moved into the screen opening of the x-ray screen unit (RBE) such that at least parts of the overlapping areas of partial x-ray images (RBn-x,..., RBn, ...,RBn+y) to be joined to one another can be created using a reduced x-ray dose, wherein the width of an overlapping area (ÜB) between two adjoining partial x-ray images (RBn-1, RBn; RBn, RBn+1) can be predetermined.

2. Device according to claim 1,
**characterised in that**
an input unit (E, B, TE) is provided with which a first boundary line (AP) and a second boundary line (EP) can be marked on a patient (P) represented on a monitor element (B, TE) .

3. Device according to claim 2,
**characterised in that**
at least one focus area (FB) can be predetermined.

4. Device according to one of the preceding claims,
**characterised in that**
it has an image computer (BR), wherein the segmentation of the partial x-ray images (RBn-x, ..., RBn, ..., RBn+y) for a panoramic x-ray recording (RPA) can be specified with the aid of a camera (KA) recording the patient (P).

5. Method for operating a device with an x-ray source (RQ) having an x-ray screen unit (RBE) and a detector (D) for creating partial x-ray images (RBn-1, RBn, ..., RBn+1),
wherein
when partial x-ray images (RBn-1, RBn, ..., RBn+1) are created for a panoramic x-ray recording (RPA), a first and/or second semi-transparent collimator (ERB, ZRB) is moved into the screen opening of the x-ray screen unit (RBE) such that at least parts of the overlapping areas of partial x-ray images (RBn-1, RBn, ...,RBn+1) to be joined to one another are created using a reduced x-ray dose and the width of the overlapping areas (ÜB) between two adjoining partial x-ray images (RBn-1, RBn; RBn,RBn+1) is predetermined.

6. Method according to claim 5,
**characterised in that**
a first boundary line (AP) and a second boundary line (EP) can be marked on a patient (P) represented on a monitor element (B, TE).

7. Method according to one of claims 5 or 6,
**characterised in that**
at least one focus area (FB) is specified.

8. Method according to one of the preceding claims 5 to 7,
**characterised in that**
the segmentation of the partial x-ray images (RBn-x,...,RBn,...,RBn+y) for a panoramic x-ray recording (RPA) is created on the basis of a camera (KA) recording the patient (P) .

## Revendications

1. Installation comprenant une source (RQ) de rayons X, ayant une unité (RBE) de diaphragme de rayons X, et un détecteur (D) pour l'établissement d'images (RBn-x, ..., RBn,..., RBn+y) partielles de rayons X,
dans laquelle l'unité (RBE) de diaphragme de rayons X ayant un premier et/ou un deuxième collimateur (ERB, ZRB) semi-transparent, peut, lors de l'établissement d'images (RBn-x,..., RBn,..., RBn+y) partielles de rayons X d'un enregistrement (RPA) panoramique de rayons X, être commandée de manière à ce que, lors de l'établissement d'images (RBn-x,..., RBn,..., RBn+y) partielles de rayons X pour un enregistrement (RPA) panoramique de rayons X, le premier et/ou le deuxième collimateur (ERB, ZRB) semi-transparent soient déplacés dans l'ouverture de diaphragme de l'unité (RBE) de diaphragme de rayons X de manière en ce qu'au moins des parties des régions en chevauchement d'images (RBn-x,..., RBn,..., RBn+y) partielles de rayons X à juxtaposer puissent être établies avec une dose de rayons X réduite, la largeur d'une région (ÜB) de chevauchement entre deux images (RBn-1, RBn; RBn, RBn+1) partielles de rayons X voisines pouvant être donnée à l'avance.

2. Installation suivant la revendication 1,
**caractérisée**
**en ce qu'**il est prévu une unité (E, B, TE) d'entrée, par laquelle une première ligne (AP) de démarcation et une deuxième ligne (EP) de démarcation peuvent être repérées sur un patient (P) représenté sur un élément (B, TE) d'écran.

3. Installation suivant la revendication 2,
**caractérisée**
**en ce qu'**au moins une région (FB) focale peut être donnée à l'avance.

4. Installation suivant l'une des revendications précédentes,
**caractérisée**
**en ce que** celle-ci a un ordinateur (BR) d'images, la subdivision des images (RBn-x, ..., RBn, ..., RBn+y) partielles de rayons X pour un enregistrement (RPA) panoramique de rayons X pouvant être indiquée à l'aide d'un appareil (KA) photographique enregistrant le patient (P).

5. Procédé pour faire fonctionner une installation comprenant une source (RQ) de rayons X, ayant une unité (RBE) de diaphragme de rayons X et un détecteur (D) pour l'établissement d'images (RBn-1, RBn, ..., RBn+1) partielles de rayons X, dans lequel, lors de l'établissement d'images (RBn-1, RBn, ..., RBn+1) partielles de rayons X pour un enregistrement (RPA) panoramique de rayons X, on déplace un premier et/ou un deuxième collimateur (ERB, ZRB) semi-transparent dans l'ouverture de diaphragme de l'unité (RBE) de diaphragme de rayons X de manière à ce qu'au moins des parties des régions qui se chevauchent d'images (RBn-1, RBn, ..., RBn+1) partielles de rayons X à juxtaposer soient établies avec une dose de rayons X réduite et on prescrit la largeur des régions (ÜB) de chevauchement entre deux images (RBn-1, RBn, ..., RBn+1) partielles de rayons X voisines.

6. Procédé suivant la revendication 5,
**caractérisé**
**en ce qu'**une première ligne (AP) de démarcation et une deuxième ligne (EP) de démarcation peuvent être repérées sur un patient (P) représenté sur un élément (B, TE) d'écran.

7. Procédé suivant l'une des revendications 5 ou 6,
**caractérisé**
**en ce qu'**on indique au moins une région (FB) focale.

8. Procédé suivant l'une des revendications 5 à 7 précédentes,
**caractérisé**
**en ce qu'**on établit la répartition des images (RBn-x, ..., RBn, ..., RBn+y) partielles de rayons X pour un enregistrement (RPA) panoramique de rayons X à l'aide d'un appareil (KA) photographique enregistrant le patient (P).
